(19) Europäisches Patentamt
European Patent Office
Office européen des brevets

(11) **EP 3 130 679 B1**

(12) **EUROPÄISCHE PATENTSCHRIFT**

(45) Veröffentlichungstag und Bekanntmachung des Hinweises auf die Patenterteilung:
**28.02.2018 Patentblatt 2018/09**

(51) Int Cl.:
*C12Q 1/68* (2018.01)   *G06F 19/24* (2011.01)

(21) Anmeldenummer: **15180991.0**

(22) Anmeldetag: **13.08.2015**

(54) **VERFAHREN UND TESTSYSTEM ZUM NACHWEIS UND/ODER QUANTIFIZIEREN EINER ZIEL-NUKLEINSÄURE IN EINER PROBE**

METHOD AND TEST SYSTEM FOR THE DETECTION AND/OR QUANTIFICATION OF A TARGET NUCLEIC ACID IN A SAMPLE

PROCEDE ET SYSTEME D'ESSAI DESTINES A LA VERIFICATION ET/OU LA QUANTIFICATION D'UN ACIDE NUCLEIQUE CIBLE DANS UN ECHANTILLON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**15.02.2017 Patentblatt 2017/07**

(73) Patentinhaber: **Cladiac GmbH**
**69123 Heidelberg (DE)**

(72) Erfinder:
• **ZIEGLER, Simon**
**69124 Heidelberg (DE)**
• **KEIMER, Simon**
**69181 Leimen (DE)**
• **STÜLLEIN, Christian**
**64560 Riedstadt (DE)**
• **ROS, Alexandra**
**76287 Rheinstetten (DE)**

(74) Vertreter: **Patentanwälte Dr. Keller, Schwertfeger**
**Westring 17**
**76829 Landau (DE)**

(56) Entgegenhaltungen:
EP-A2- 1 288 314      WO-A1-2014/022827
US-A1- 2009 037 117      US-A1- 2014 278 126

• **PORNPAT ATHAMANOLAP ET AL: "Trainable High Resolution Melt Curve Machine Learning Classifier for Large-Scale Reliable Genotyping of Sequence Variants", PLOS ONE, Bd. 9, Nr. 10, 2. Oktober 2014 (2014-10-02), Seiten e109094-1, XP55233770, DOI: 10.1371/journal.pone.0109094**

**Beschreibung**

Die vorliegende Erfindung betrifft ein Verfahren zum Nachweis und/oder Quantifizieren einer Ziel-Nukleinsäure in einer Polymerase-Kettenreaktion (PCR)-Probe und ein dafür entwickeltes Testsystem zur Durchführung einer symmetrischen oder asymmetrischen Polymerase-Kettenreaktion (PCR). Das Verfahren eignet sich beispielsweise zum Nachweis von Mutationen in einer Nukleinsäuresequenz, von pathogenen Erregern oder von Gensequenzen.

Die Polymerase-Kettenreaktion (polymerase chain reaction, PCR) ist ein übliches Verfahren zur Vervielfältigung und zum spezifischen Nachweis von Nukleinsäuren, wie z.B. DNA oder RNA (Mulis K et al., Specific enzymatic amplification of DNA in vitro: the polmerase chain reaction. Cold Spring Harb Symp Quant Biol 1986, 51 Pr I: 263-273). Die PCR ist, auch in ihren verschiedenen Varianten, eine polyzyklische Reaktion, die zumindest zwei, in der Ursprungsvariante drei Schritte erfordert. Beim ersten Schritt wird die DNA thermisch hybridisiert (d.h. denaturiert), was bei hohen Temperaturen erfolgt. Im nächsten Schritt erfolgt bei einer niedrigeren Temperatur die Anlagerung (Annealing) von Oligonukleotid-Primern (z.B. Sonden) an die Ziel-Nukleinsäure, was auch als Primerhybridisierung bezeichnet wird. Ausgehend von den Primern findet in einem weiteren Schritt die Vervielfältigung bzw. Synthese eines Komplementärstranges durch eine thermostabile Polymerase (z.B. DNA-Polymerase) statt. Die für die PCR-Reaktion eingesetzten Primer bestehen üblicherweise aus kurzen Basenpaar-Segmenten mit Längen zwischen 15 und 40 Basenpaaren, die komplementär zur Zielsequenz sind. Die PCR besteht demnach aus einer Reaktionsabfolge aus Denaturierung, Annealing und Synthese.

Ein typisches PCR-Reaktionsprofil umschreibt drei Phasen, nämlich eine frühe Lag-Phase, eine exponentielle Wachstumsphase und eine Plateau-Phase. Die Lag-Phase spiegelt im Wesentlichen die Empfindlichkeit der Synthesevorrichtung und das Rauschsignal des für den Nachweis des PCR-Produktes verwendeten Sondensystems wider. Mit fortschreitenden PCR-Zyklen und ausreichender Produkt-Akkumulation beginnt die exponentielle Wachstumsphase. In der exponentiellen Wachstumsphase findet sich auch die meiste quantitative Information zum Nachweis einer Ziel-Nukleinsäure.

Aus diesem PCR-Standard haben sich davon abgeleitete Methoden entwickelt, wie beispielsweise die Multiplex-PCR (MPCR; Henegariu et al., Multiplex PCR: critical parameters and step-by-step protocol, 1997, 23: 504-511), reverse Transkriptase-PCR (RT-PCR; Mothershed EA, Whitney AM: Nucleic acid-based methods for the detection of bacterial pathogens: present and future considerations for the clinical laboratory. Clin Chim Acta 2006, 363: 206-220) und quantitative Multiplex-Echtzeit-PCR (qPCR) (Heid CA et al., Real time quantitative PCR. 1996, 6: 986-994; Espy MJ et al., Real-time PCR in clinical microbiology: applications for routine laboratory testing. 2006, 19:165-256; Wittwer CT et al., Real-time multiplex PCR assays. Methods 2001, 25: 430-442; Mackay IM et al., Real-time PCR in virology. Nucleic Acids res 2002, 30: 1292-1305).

Je nach Anwendungsgebiet eignet sich die jeweilige PCR-Methode zum Nachweis von Markergenen oder zur Quantifizierung von Genprodukten, beispielsweise im Rahmen der Untersuchung der Genregulation.

In Abhängigkeit davon, in welchen Oligonukleotid-Verhältnissen die Primer vorliegen, unterscheidet man zwischen einer symmetrischen PCR und einer asymmetrischen PCR. Bei einer symmetrischen PCR liegen die Vorwärts- und Rückwärts-Primer in einem äquimolaren (gleichen) Verhältnis vor, so dass die Nukleinsäure-Stränge bei jedem PCR-Zyklus verdoppelt werden. Nach der Denaturierung binden Vorwärts- und Rückwärts-Primer an die Einzelstränge und werden durch die Polymerase vervollständigt, bevor sie im nächsten Zyklus wieder denaturiert werden. Die für die Reaktion notwendigen Bestandteile werden Zyklus für Zyklus exponentiell aufgebraucht, so dass die Reaktion schließlich in der Plateauphase endet. Die PCR-Reaktion zeigt einen sigmoiden Reaktionsverlauf, beginnend mit der Lag-Phase und einer sich anschließenden Log-linearen Phase. Sobald die für die PCR-Reaktion erforderlichen Bestandteile aufgebraucht sind, kommt es zu einem Abflachen der Amplifikationskurve und zum Übergang in die Plateauphase. Dieses Plateau korreliert allerdings nicht mit der Menge der Zielmoleküle in der Lösung (Hartshorn C et al, Single-cell duplex RT-LATE-PCR reveals Oct4 and Xist RNA gradients in 8-cell embryos. BMC Biotechnol. 2007, 7: 87). Die klassische PCR ermöglicht deshalb nur qualitative Aussagen, was letztendlich die Grundlage für die sich daraus entwickelte asymmetrische PCR war, mit der quantitative Messungen durchgeführt werden können (Gyllensten UB, Erlich HA: Generation of single-stranded DNA by the polymerase chain reaction and ist application to direct sequencing oft he HLA-DQA locus. Proc Natl Acad Sci U S A 1988, 85: 7652-7656).

Im Gegensatz zu einer symmetrischen PCR liegen bei einer asymmetrischen PCR nichtäquimolare Verhältnisse der Primer vor, d.h. in der Reaktion wird ein limitierender Primer und ein Überschuss-Primer verwendet (Gyllensten UB, Erlich HA: Generation of single-stranded DNA by the polymerase chain reaction and ist application to direct sequencing oft he HLA-DQA locus. Proc Natl Acad Sci U S A 1988, 85: 7652-7656). Eine asymmetrische PCR zeigt einen typischen Log-linearen Verlauf, wobei sich der lineare Verlauf einstellt, sobald der limitierende Primer in der Reaktion verbraucht wurde. In dieser Phase wird einer der beiden Stränge arithmetisch amplifiziert, was zur Folge hat, dass asymmetrische PCR-Verfahren zumeist eine niedrige Amplifikationseffizienz gegenüber symmetrischen PCR-Verfahren haben. Allerdings weist eine asymmetrische, quantitative PCR ein besser analysierbares Schmelzkurvenverhalten auf, was beispielsweise bei der Mutationsdetektion oder Allel-Bestimmung vorteilhaft ist (Szilvási A. et al., Asymmetric PCR increases efficiency of melting peak analysis on the LightCycler. Clin Biochem 2005, 38: 727-730). Ein weiterer Vorteil der asymmetrischen PCR besteht darin, dass die Nukleinsäure-Einzelstränge bis auf die Primer-Hybridisierungsstelle für Sonden in qPCR- Systemen frei zugänglich sind und dass Einzelstränge nicht durch einen Schmelzpunkt definiert sind, was die Bedingungen bei der Primer-Auswahl und Primer-Hybridisierung verbessert. Dabei können die Sonden bis zur Sättigungskonzentration der Einzelstränge verwendet werden (Sanchez JA et al., Linear-after-the-exponential (LATE)-PCR: an advanced method of asymmetric PCR and its uses in quantitative real-time analysis. Proc Natl Acad Sci U S A 2004, 101: 1933-1938; Sanchez JA et al., Two-temperature LATE-PCR endpoint genotyping. BMC Biotechnol 2006, 6: 44).

Daneben existieren kompetitive quantitative PCR-Verfahren, die entwickelt wurden, um das Auffinden der exponentiellen Phase beim Kurvenverlauf zu vereinfachen und um eine größere Genauigkeit zu erzielen. Kompetitor und Target werden normalerweise durch die Länge oder Sequenz unterschieden, wobei die relativen Mengen des Kompetitors und des Targets nach der Amplifikationsreaktion gemessen werden. Problematisch ist hierbei, dass der Kompetitor zu der unbekannten Probe in einer ähnlichen Konzentration hinzugefügt werden muss wie die des Targets.

Quantitative Echtzeit-PCR-Verfahren (real-time PCR) ermöglichen eine relativ genaue Quantifizierung einer Ziel-Nukleinsäure in einer Probe. Ein prominentes Beispiel einer solchen quantitativen PCR ist das Lightcycler® -System (Roche Diagnostics), bei dem ein PCR-Produkt über fluoreszierende Farbstoffe detektiert und ausgewertet werden können. Basierend auf der asymmetrischen PCR wurde das LATE (Linear After The Exponential)-PCR-Verfahren entwickelt, bei dem ebenfalls unterschiedliche Primer-Konzentrationen eingesetzt werden, wobei der limitierende Primer jedoch so modifiziert ist, dass sein Schmelzpunkt bei einer höheren Temperatur liegt als die Schmelztemperatur des überschüssigen Primers. Dadurch kann der Vorgang der Vervielfältigung von der durch Sonden vermittelten Messung entkoppelt werden. Die Sondenentwicklung kann insbesondere dadurch vereinfacht werden, dass Sonden mit sehr tiefem Schmelzpunkt eingesetzt werden können, so dass die LATE-PCR eine weit höhere Sensitivität aufweist als übliche symmetrische oder asymmetrische PCR-Methoden. Bedingt wird dies durch eine kurze exponentielle und eine sich anschließende lineare Amplifikationsphase ohne Ausbildung eines Plateaus (Sanchez JA et al., Linear-after-the-exponential (LATE)-PCR: an advanced method of asymmetric PCR and its uses in quantitative real-time analysis. Proc Natl Acad Sci U S A 2004, 101: 1933-1938).

Eine typische LATE-PCR läuft so ab, dass sich die Anzahl der Kopien exponentiell pro Zyklus erhöht, ähnlich wie bei einer symmetrischen Reaktion. Die experimentale Phase dauert nur so lange an, bis der begrenzende Primer, der bei einer höheren Schmelztemperatur bindet als der überschüssige Primer, in der PCR-Reaktion noch vorhanden ist. Ist der begrenzende Primer aufgebraucht, werden nur noch Einzelstränge kopiert. Idealerweise ist der begrenzende Primer so entwickelt, dass er gerade dann aufgebraucht ist, wenn die untere Detektionsgrenze erreicht ist. Da der Komplementärstrang in dieser Phase nicht mitkopiert wird, kann mit Sonden mit tiefem Schmelzpunkt gearbeitet werden, was letztendlich auch dazu führt, dass die Reaktion nicht in die Plateauphase läuft. Für eine Weiterverarbeitung (z.B. DNA-Sequenzierung, SNP- Genotypisierung) ist zudem das Vorliegen von Einzelsträngen vorteilhafter.

Schon früh hat man erkannt, dass eine Schmelzkurvenanalyse der bei der PCR-Reaktion erhaltenen Schmelzkurven wertvolle quantitative Informationen bezüglich einer Ziel-Nukleinsäure liefern kann. Gerade in komplexen (Multiplex-)Systemen ist eine akkurate Schmelzkurvenanalyse für eine empfindliche und genaue Quantifizierung der in einer Probe erhaltenen Ziel-Nukleinsäuren unabdingbar. So beschreibt bereits die EP 1 375 674 B1 ein Verfahren zum Nachweis von Nukleinsäuren auf Basis einer Schmelzkurvenanalyse, bei der es möglich ist, wenigstens zwei Nukleinsäuren oder mehr mit einem einzigen Fluoreszenzfarbstoff-Marker nachzuweisen). Dabei werden die Ziel- Nukleinsäuren auf Basis ihrer Schmelztemperaturen (Tm) nachgewiesen, ohne dass eine Vielzahl von Oligonukleotid-Sonden mit unterschiedlichen Fluoreszenzfarbstoffen hergestellt werden muss. Das Verfahren umfasst das gleichzeitige Nachweisen von RNA- Transkripten in einer Probe auf der Basis unterschiedlicher TM-Werte der Duplexe, wobei die Nukleinsäure-Sonden so gewählt sind, dass sie ihre Fluoreszenzintensitäten ändern, wenn sie Duplexe mit den sich ergebenden Transkripten ausbilden.

In der EP 1 942 196 B1 wird das LATE-PCR-Verfahren beschrieben, bei dem unterschiedliche Schmelztemperaturen der eingesetzten Primer zur Anwendung kommen, wobei der Schmelzpunkt des limitierenden Primers wenigstens 7 bis 15° C höher liegt als die des Überschussprimers. Idealerweise soll dabei die Schmelztemperatur des limitierenden Primers nicht höher liegen als 18° C gegenüber dem Überschussprimer. Dagegen sind auf mehreren Primer-Paaren basierende Multiplex-PCRs sowohl in der Gestaltung der Reaktionsbedingungen als auch in der Primerauswahl weit aufwändiger (vgl. beispielsweise DE 10 2007 041 864 B4). Die DE 10 2007 031 137 A1 beschreibt ein Echtzeit-PCR-Verfahren, bei dem eine Schmelzkurve aufgezeichnet und nach der Amplifikation einer Schmelzkurvenanalyse unterzogen wurde.

Die EP 1 288 314 B1 bestimmt die Schmelzkurven für jede einzelne Ziel-Nukleinsäure, indem ein Signalverarbeitungs-Algorithmus verwendet wird, der auf einer Thermodynamik basiert. Dadurch können die molaren Verhältnisse der Zielnukleinsäuren bestimmt werden. Hierzu wird eine Fluoreszenzveränderung aufgezeichnet, wobei folgender Zusammenhang bei den daraus resultierenden Schmelzkurven besteht:

$$\int_{T_0}^{T_1} \left\| f_{\min}^{\sigma} - \sum_i m_i f_i^{\sigma}(T) - f^r(T) \right\| dT$$

$\Sigma$ = Glättungsparameter
$m_i$ = Massenfraktion von jedem Nukleinsäure-Target
$f^r$ = die angenäherte Fluoreszenz der Schmelzkurve, wobei die Optimierung und der iterative Prozess wiederholt werden, bis eine Summe der Massenfraktion größer 1 minus $\varepsilon$ ist, wobei $\varepsilon$ ein Toleranzwert ist.

Voraussetzung des ebenfalls darin beschriebenen Quantifizierungsverfahrens ist, dass die Effizienz der Amplifikation für das Target und den Kompetitor im Wesentlichen gleich ist, wobei $\log C_O = \log E (\Delta n) + \log T_O$, wobei $C_O$ die initiale Kompetitormenge ist, E die durchschnittliche Effizienz ist, $\Delta n$ die Zyklusverschiebung zwischen Target und Kompetitor ist und $T_O$ die initiale Menge des Targets ist.

Durch das Auftragen der initialen Kompetitorkonzentration gegenüber der Zyklusverschiebung zwischen Kompetitor und Target ergibt sich ein Kurvenverlauf mit einer Steigung, die dem Log der Effizienz entspricht und ein Log, welcher der initialen Targetkonzentration entspricht. Dadurch die initiale Konzentration des Targets ermittelt werden.

Obgleich die Reaktionsbedingungen zum Durchführen einer symmetrischen oder asymmetrischen PCR durch eine geschickte Auswahl der Primer unter Berücksichtigung der Schmelztemperaturen umfassend beschrieben sind, wurde bislang wenig Augenmerk auf eine Optimierung der Schmelzkurvenanalyse im Rahmen einer PCR-Reaktion gelegt. Dabei verbergen sich im Kurvenverlauf einzelner Schmelzkurven wertvolle Informationen, die es ermöglichen, Detektionseffizienzen bei der Bestimmung von Ziel-Nukleinsäuren zu erhöhen. Häufig kamen nur übliche Berechnungsmethoden zum Einsatz, um beispielsweise den Beginn einer Steigung im Kurvenverlauf einer Schmelzkurve zu bestimmen. Solche Verfahrensweisen sind insbesondere in bi-allelischen Systemen nicht ausreichend, da diese eine weit höhere Genauigkeit und Effizienz erfordern. Dabei kommen Algorithmen zum Einsatz, die auf einer thermodynamischen Modellierung basieren, d.h. Fourier-Transformationen mit Fourier-Modi mit geringer Amplitude beim Rauschen in einem Signalpunkt. Das thermodynamische Modeling basiert auf der frei werdenden Gibbs-Energie einer Mischung und nimmt an, dass es keine chemische Interaktion zwischen geschmolzenen Materialien gibt. Der zugrundeliegende Algorithmus beinhaltet zusätzlich die Eigenschaft, das Schmelzsignal einer chemischen Probe in Abwesenheit von Standards zu analysieren.

Bisherige Analyseverfahren zielen auf die Verwendung einer Fourier-basierten D-Konvolution ab, wie beispielsweise in den US-Patenten Nr. 5,273,632, 5,748,491 und 5,346,306 beschrieben. Die US 316 614 P beschreibt darauf aufbauend eine Signalverarbeitung, basierend auf einer diskreten Fourier-Transformation (DFT), die darauf abzielt, das Signal als eine lineare Kombination sinusoidaler Signale auf Basisfunktion darzustellen und nur solche sinusoidalen Basisfunktionen zu behalten, die verlässliche Informationen über das Signal enthalten. Hierbei wird eine Basisfunktionsapproximierung und ein Koppeln der Algorithmen vorgenommen. Der Approximierungsalgorithmus nimmt eine Schmelzkurve und trennt diese in zwei Standardkurven auf, um die Verhältnisse in unbekannten Proben unter Verwendung des TMBSP-Algorithmus zu bestimmen. Dieses Verfahren ist jedoch nicht optimal, da der Kurvenverlauf bei niedrigen Detektionsgenauen in einer PCR-Relation nicht exakt bestimmbar ist. Die WO2014/022827 beschreibt ein Verfahren zum Nachweis und zur Quantifizierung von Zielnukleinsäuren, welches eine Schmelzkurvenanalyse umfasst, die einzelne "Peaks" analysiert, wobei die Analyse jedoch nicht auf Merkmalsvektoren und auch nicht auf einer Klasseneinteilung von Merkmalen durch gewichtete Klassifikatoren beruht. Vor diesem Hintergrund ist es Aufgabe der vorliegenden Erfindung, ein optimiertes Verfahren und ein Testsystem zum Nachweis und/oder Quantifizieren einer Ziel-Nukleinsäure in einer Polymerase-Kettenreaktion (PCR)-Probe, basierend auf einer Schmelzkurvenanalyse, bereitzustellen, um eine erhöhte Detektionseffizienz zu erhalten. Diese Aufgabe wird gelöst durch ein Verfahren mit den Merkmalen des Anspruchs 1 bzw. ein Testsystem mit den Merkmalen des Anspruchs 12. Bevorzugte Ausführungsformen finden sich in den Unteransprüchen wieder.

Das erfindungsgemäße Verfahren basiert auf einer optimierten Schmelzkurvenanalyse unter Anwendung mathematischer Methoden, die automatisiert über eine Steuerungs- und Regeleinrichtung ablaufen können. Die technische Umsetzung sieht eine Analyse- und Auswerteeinheit vor, bei der die im Rahmen der Amplifikationsreaktionen gewonnenen Rohdaten zur Generierung der Schmelzkurven in optimierte Schmelzkurvenmerkmale umgewandelt werden, um so den Reaktionsverlauf und das Reaktionsverhalten einer PCR-Reaktion nachzuvollziehen und gegebenenfalls zu kontrollieren.

Ausgangsbasis ist ein übliches PCR-Reaktionsgemisch, wobei die Anwendbarkeit des Verfahrens sich nicht auf eine bestimmte PCR-Methode beschränkt, sondern für alle gängigen symmetrischen, asymmetrischen und LATE-PCR-Methoden sowie Kombinationen einzelner PCR-Methoden geeignet ist. Gängige PCR-Verfahren sehen den Einsatz einer doppelsträngigen Nukleinsäure (DNA, Desoxynukleosid-Triphosphate (dNTPs)) sowie einer DNA-Polymerase vor. Beispielhafte, in der Erfindung verwendbare DNA-Polymerasen, sind die Taq-DNA-Polymerase oder andere thermostabile DNA-Polymerasen. Ferner ist eine Ziel-Nukleinsäure mit einer zu amplifizierenden Nukleinsäure-Sequenz notwendig. Als SyntheseBausteine dienen üblicherweise Nukleosid-Triphosphate (NTTs), vorzugsweise Desoxynukleosid-Triphosphate (dNTPs wie dATT, dTTT, dGTT, gCTP). Üblicherweise wird eine PCR-Reaktion in einer Pufferlösung durchgeführt, die geeignete Reaktionsbedingungen und stabile Verhältnisse für eine DNA-Synthese durch die PNA-Polymerase bietet. Das PCR-Gemisch kann dann beispielsweise bestimmte Kationen oder Anionen enthalten, welche die Aktivität und Stabilität der DNA-Polymerase erhöhen. Ferner ist wenigstens ein zu der amplifizierenden Nukleinsäure- Zielsequenz komplementäres Oligonukleotid-Primerpaar erforderlich, dessen Primer unter PCR-Bedingungen an den zu amplifizierenden Bereich der Nukleinsäure-Zielsequenz hybridisieren. Für dieses Reaktionsgemisch werden mehrere Amplifikationszyklen (beispielsweise 20 bis 40 PCR-Zyklen) der Polymerase-Kettenreaktion mit einer PCR-Probe durchgeführt, um die für die weitere Analyse erforderlichen Schmelzkurven zu erhalten. Aus den erhaltenen Schmelzkurven werden Signale generiert und der nachgeschalteten erfindungsgemäßen Schmelzkurvenanalyse unterzogen.

Hierfür werden zunächst anhand der Stützpunkte der Schmelzkurve die negativen Steigungen durch numerische Ableitung bestimmt. Gegebenenfalls kann die erzeugte erste numerische Ableitung der Kurve mit einem Filter geglättet werden. Hierfür eignen sich beispielsweise ein Median- oder Gaussfilter.

Anschließend werden bestimmte Merkmale der Kurve ermittelt, die die sogenannten Merkmalsvektoren bilden. Eine Variante ist die Merkmalsgewinnung mittels Fouriertransformation. Hierbei wird die Schmelzkurve in ihre Spektralkomponenten zerlegt und sowohl Real- als auch Imaginär-Teil der dabei erhaltenen komplexen Koeffizienten übernommen, welche Informationen über Amplitude und Phase der Spektralkomponenten beinhalten. Ein Teil der Koeffizienten wird zur Bildung des Merkmalsvektors der betrachteten Kurve verwendet.

Eine weitere Art der Merkmalsgewinnung ist die Wavelet-Analyse. Die Wavelet-Analyse bezeichnet den Übergang der Zeitdarstellung in die Spektral- bzw. Wavelet-Darstellung, während die Wavelet-Synthese die Rücktransformation der Wavelet-transformierten in die Zeitdarstellung bezeichnet. Dabei wird das zu untersuchende Signal mit einer Fensterfunktion verglichen. Anstatt allerdings das Fenster zu verschieben oder zu modulieren (d.h. durch Verschiebung im Frequenzbereich), wird das Fenster verschoben und skaliert. Durch die Skalierung ergibt sich zwar eine Frequenzverschiebung, wie bei der Modulation, jedoch wird gleichzeitig mit der Frequenzerhöhung die Zeitdauer des Fensters verringert. Dadurch ergibt sich bei höheren Frequenzen eine weitaus bessere zeitliche Auflösung, während bei niedrigen Frequenzen die Frequenzauflösung verbessert wird, dafür die Zeitauflösung schlechter.

Zudem werden sogenannte "einfache" Merkmale zur Erzeugung von Merkmalsvektoren verwendet. Dies sind z.B. die Position (x- und y-Wert) der lokalen Extremwerte der Kurve, ergänzt mit Fourierkoeffizenten aus der Fouriertransformation.

Alle drei Arten von Merkmalsvektoren (Fourier, Wavelet und "einfache") können unabhängig voneinander von folgenden Klassifikatoren verwendet werden: Support Vector Maschine, Lernende Vektor-Quantisierung und Random Forest. Maschinelle Lernverfahren entwickeln eine Generalisierungsfähigkeit, mit der unbekannte Merkmalsvektoren einer von mehreren Klassen zugeordnet werden können. Dies ergibt dann insgesamt neun Klassifikatoren. Die Erzeugung von Merkmalen soll das Problem des "Overfittings" vermeiden. "Overfitting" ist eine Überanpassung des Klassifikators auf die Trainingsmenge und kann als "Auswendiglernen" interpretiert werden, welches eintritt, wenn die Merkmalsvektoren eine zu hohe Anzahl von Elementen besitzen. In diesem Fall kann das maschinelle Lernverfahren keine Generalisierungsfähigkeit ausbilden. Die Generalisierungsfähigkeit kann mit einer Testmenge (die keine Teilmenge der Trainingsmenge ist) bestimmt werden.

Grundsätzlich werden Algorithmen für maschinelles Lernen (Klassifikatoren) mit einer Trainingsmenge von Merkmalsvektoren trainiert. Eine Testmenge dient der Bewertung eines trainierten Klassifikators. Hierbei ist es wichtig, für jede Klasse mehrere repräsentative Kurven zu finden, um später unbekannte Kurven sicher und korrekt zu klassifizieren. Die Verwendung verschiedener Klassifikatoren verbessert das Gesamtergebnis. Jeder Klassifikator gibt eine Stimme ab, zu welcher Klasse ein Merkmalsvektor gehört. Die Klasse mit den meisten Stimmen "gewinnt". Die Stimmen können auch gewichtet werden.

Erfindungsgemäß können die im Folgenden beschriebenen maschinellen Lernverfahren als Klassifikator eingesetzt werden.

Als erster Klassifikator kommt eine Support Vector Machine (SVM) zum Einsatz, die in ihrer einfachsten Form ein binärer Klassifikator ist, der ein Objekt in eine von zwei möglichen Klassen einordnen kann. Um mehr als zwei Klassen unterscheiden zu können verwendet man z.B. die "one vs one" Methode. Es werden in allen möglichen Kombinationen immer zwei verschiedene Klassen gegeneinander getestet. Aus jedem dieser Tests wird für eine der Klassen eine Stimme vergeben. Die Klasse mit den meisten Stimmen wird als Klassifikationsergebnis gewertet.

Intern arbeiten Support Vector Machines mit sogenannten "Hyperebenen", welche die verschiedenen Klassen voneinander trennen. Diese Hyperebenen werden beim Training der SVM dermaßen bestimmt, dass der Rand, d.h. der Abstand zwischen der Ebene und den einzelnen "Objekten" (Merkmalsvektoren der Trainingskurven) der Klassen maximiert wird.

Abstrakt formuliert wird versucht, eine Hyperebene derart durch einen mehrdimensionalen Raum (Anzahl der Dimensionen wird durch die Anzahl der Elemente der Merkmalsvektoren bestimmt) zu legen, dass die Merkmalsvektoren, die zu unterschiedlichen Klassen gehören, möglichst gut voneinander getrennt werden. Allgemein kann eine Ebene durch ihren Normalenvektor w (steht senkrecht auf der Ebene) und ein Offset b definiert. Damit kann man die Klassenzugehörigkeit y eines Vektors x über folgende Gleichung ermitteln:

y = sgn (<w,x>+b), dabei entspricht Klasse A: y = +1, Klasse B: y = -1.

Dabei ist <w,x> das Skalarprodukt zwischen dem Normalenvektor w und dem Vektor x, und sgn() die Vorzeichenfunktion. Wenn x nun auf der einen Seite der Ebene ist, kommt ein positivies Ergebnis heraus. Wenn x auf der anderen Seite ist erhält man ein negatives Ergebnis, so dass über das Vorzeichen zwischen den zwei Klassen unterschieden werden kann. Hierbei ist ersichtlich, dass nicht jeder Vektor zi bedeutend ist, sondern lediglich diejenigen, die der Hyperebene am nächsten sind (Stützvektoren). Es wird also ein w und b gesucht, so dass für die Vektoren zi gilt:

$$\min_{z_i} |<w,z_i> + b| = 1 \quad |<w,z>+b|=1$$

.

Für Punkte, die weiter von der Hyperebene entfernt sind, kann dieser Wert größer sein, aber er soll für die gegebenen (Trainings-)Daten betraglich nie kleiner als 1 sein.

Beim Training der SVM werden die Parameter w und b so optimiert, dass der Rand der Hyperebene (Abstand zu den Support Vektoren) maximal wird.

Im Allgemeinen sind Klassen nicht so leicht linear trennbar wie im gezeigten Beispiel. In den nachfolgenden Ausführungsbeispielen ist eine komplexere Verteilung der Klassenobjekte bei Anwendung der SVM dargestellt.

Um die Hyperebene zu deformieren, wird ein sogenannter Kernel-Trick angewendet. Hierbei werden die Skalarprodukte <w,x> durch eine Kernelfunktion K(w,x) ersetzt. Die Klassenzugehörigkeit y eines Merkmalsvektors x ist über eine Kernel-Funktion wie folgt ermittelbar: y = sgn(K(w,x)+b).

Deformierte Hyperebenen sind notwendig, wenn Merkmalsvektoren verschiedener Klassen nicht linear trennbar sind, also nicht trennbar durch eine Hyperebene. Dies ist in der Regel der Fall. Die Bestimmung der Parameter der Kernelfunktion wird anhand der Testmenge vorgenommen, wobei die Parameter ausprobiert werden. Die Parameter, für die die Testmenge am besten klassifiziert wird, werden verwendet.

Das zweite maschinelle Lernverfahren ist die Lernende Vektor-Quantisierung (LVQ). Hierbei handelt es sich um eine Form eines künstlichen neuronalen Netzwerkes. Im Rahmen des Trainings werden sogenannte "Neuronen" in dem Merkmalsraum verteilt, wobei es für jede der n Klassen m Neuronen gibt.

In einem iterativen Verfahren wird nun nacheinander der Abstand jedes Neurons zu jedem Trainingsobjekt (Merkmalsvektor) bestimmt. Gehören das aktuell betrachtete Trainingsobjekt und das Neuron der gleichen Klasse an, so wird das Neuron in Richtung Trainingsobjekt verschoben. Anderenfalls wird das Neuron in die Gegenrichtung bewegt. Wie groß diese Verschiebungen sind, hängt von einem Parameter ab, der auch dynamisch angepasst werden kann. So können beispielsweise starke Verschiebungen zu Beginn und bei nachfolgenden Iterationen kleinere Verschiebungen vorgesehen werden. Nach einer gewissen Anzahl an Iterationen erhält man ein "angelerntes Netzwerk". Dabei können verschiedene Abbruchbedingungen definiert werden, beispielsweise eine maximale Anzahl an Iterationen oder Abbruch, wenn die Änderungen während einer Iteration unterhalb eines definierten Schwellwerts liegen.

Die Zuordnung eines unbekannten Objektes zu einer der Klassen erfolgt über die Ermittlung des kleinsten Abstandes der Objektmerkmale zu einem Neuron. Das Neuron, welches dem Objekt am nächsten ist, bestimmt dessen Klassenzugehörigkeit.

Da nicht jedes betrachtete Merkmal gleichbedeutend für eine korrekte Klassifizierung ist, kann die Zuordnung des einen Objektes zu einem Neuron variiert werden. Im normalen Fall wird der minimale euklidische Abstand $\sum_{i=1}^{s} (x^i - \lambda^i)_s$ verwendet (hier entspricht x dem Testobjekt und y einem Neuron), um den Abstand eines Objektes (genauer des Merkmalsvektors eines Objektes) zu einem Neuron zu bestimmen. Dies kann nun durch $\sum_{i=1}^{s} q^i (x^i - \lambda^i)_s$ ersetzt werden. Der Index i indiziert die einzelnen Komponenten der Vektoren. In dem zweidimensionalen Beispielfall sind das die Anteile in die X- und Y-Richtung des Koordinatensystems. Mittels des Faktors di (Werte zwischen 0 und 1) kann nun der Abstand von Testobjekt und Neuron für jede Richtung separat gewichtet werden. Ist di für eine Komponente (Richtung) 0, so hat der Abstand von Objekt und Neuron in diese Richtung keinen Einfluss auf die Klassifizierung mehr (dieses Merkmal könnte dann also auch komplett entfallen). Durch die Gewichtung ist es aber möglich, einzelne Merkmale nicht nur komplett zu ignorieren, sondern dessen Wichtigkeit für die Klassifizierung feingranular einzustellen.

Die Komponenten des Vektors d werden nun so bestimmt, dass die Klassifikationsperformance maximiert wird. Da die Merkmalsvektoren zumeist relativ viele Elemente beinhalten, und die Anzahl der Komponenten von d direkt damit korreliert, ist es nicht effizient möglich, alle möglichen Varianten von d zu testen, um den bestmöglichen Vektor zu ermitteln. Aus diesem Grund werden hier Optimierungsverfahren eingesetzt. Bei der vorliegenden Erfindung kommt hierfür ein "Genetischer Algorithmus" (GA) zum Einsatz. Hierbei werden "Populationen" von möglichen Distanzvektoren erzeugt und deren "Fitness" (Anzahl der korrekt klassifizierten Objekte der Testmenge) ermittelt. Durch "Kreuzung", "Mutation" und Selektion ermittelt der GA eine neue Population, die abermals getestet werden kann. Wenn auf diese Weise ein guter Distanzvektor gefunden wurde, wird dieser für den Klassifikator verwendet.

Die Qualität der Klassifikation eines Merkmalsvektors mit der LVQ kann wie folgt bestimmt werden: zunächst wird der Quotient aus der Distanz zum nächsten Neuron (welches die Klasse bestimmt) und zum nächsten Neuron einer anderen Klasse berechnet. Dieser Wert ist immer zwischen 0 und 1, je niedriger desto besser. Wenn nun von der Zahl 1 dieser Quotient subtrahiert wird, erhält man die Qualität, je höher desto besser.

Neben der SVM- und der LVQ-Methode wird die Random Forest-Methode zur Klassifizierung eingesetzt. Hierbei werden aus den gegebenen Merkmalen (Merkmalsvektor) viele verschiedene kurze Entscheidungsbäume erstellt. Dabei wird in jedem Baum eine zufällige Auswahl an Merkmalen verwendet. Wird nun ein Merkmalsvektor klassifiziert, gibt jeder Entscheidungsbaum eine "Stimme" ab, die Klasse wird durch Mehrheitsentscheid bestimmt. Je kürzer die Bäume im Random Forest sind, desto niedriger ist die Wahrscheinlichkeiten eines "overfittings".

Das erfindungsgemäße Schmelzkurvenanalyseverfahren eignet sich für beliebige asymmetrische oder symmetrische PCR-Verfahren oder eine Kombination davon. In einer bevorzugten Ausführungsform umfasst das Oligonukleotid-Primerpaar einen Überschuss-Primer und einen limitierenden Primer bei der Amplifikation. Dabei ist die Konzentration des limitierenden Primers so gewählt, dass er, sobald das Signal des Targets aus dem Untergrundrauschen hervortritt, erschöpft ist. Dabei soll die Schmelztemperatur des limitierenden Primers höher sein als die Schmelztemperatur des Überschuss-Primers. Vorzugsweise ist die Temperatur des limitierenden Primers um bis zu 18° C höher als die Schmelztemperatur des Überschuss-Primers. In einer bevorzugten Variante handelt es sich bei der Ziel-Nukleinsäure um eine cDNA aus einem Organismus, beispielsweise einem Mikroorganismus (viral, bakteriell). Das Verfahren eignet sich auch für die Analyse der Genregulation oder zum Nachweis von prokaryotischen, eukaryotischen und oder viralen Gensequenzen. Ferner eignet sich das erfindungsgemäße Verfahren zum Nachweis von Mutationen in einer Nukleinsäuresequenz. Eine erfindungsgemäße Nukleinsäuresequenz kann beispielsweise DNA und/oder RNA sein oder enthalten. Daneben sind auch modifizierte Nukleinsäuresequenzen oder Nukleinsäurederivate von der Erfindung umfasst.

Die Erfindung betrifft ferner ein Testsystem zur Durchführung einer symmetrischen oder asymmetrischen Polymerase-Kettenreaktion (PCR) für den Nachweis einer Ziel-Nukleinsäure in einer Probe, umfassend eine Ziel-Nukleinsäure mit einer zu amplifizierenden Nukleinsäure-Zielsequenz, eine thermostabile (DNA)-Polymerase, Desoxynucleosidtriphosphate (dNTPs), wenigstens ein zu der zu amplifizierenden Nukleinsäure-Zielsequenz komplementäres Oligonukleotid-Primerpaar, dessen Primer unter PCR-Bedingungen an den zu amplifizierenden Bereich der Nukleinsäure-Zielsequenz hybridisieren, eine Datenerfassungseinheit zur Erfassung der über die Amplifikationsreaktionen bei der PCR erhaltenen Schmelzkurven, eine Analyseeinheit zur Schmelzkurvenanalyse der erhaltenen Schmelzkurven durch ein wie oben beschriebenes Verfahren.

Vorzugsweise kommen bei der PCR-Reaktion Nukleinsäuresonden zum Einsatz, die ein Reporter- und/oder Quenchermolekül umfassen. Das Testsystem kann beispielsweise zur Durchführung einer Multiplex-PCR oder Reverse Transkriptase (RT)-PCR oder quantitativen-(Multiplex)-Echtzeit-PCR (qPCR) oder (Multiplex)-LATE-PCR verwendet werden.

Aufgrund der Sensitivität und Detektionseffizienz eignet sich das erfindungsgemäße Verfahren zur Detektion von Alllelen oder Mutationen, vorzugsweise Einzelmlutationen, in einer Nukleinsäuresequenz. Die Erfindung betrifft daher auch ein Verfahren zum Nachweis von Mutationen in einer solchen Nukleinsäuresequenz, bei dem eine Probe in Anwesenheit von zu einem Bereich der Nukleinsäure-Sequenz spezifischen Sonden mit einer bereits amplifizierten Nukleinsäure-Zielsequenz erhitzt und anschließend schrittweise abgekühlt wird, während eine Erfassung des Schmelzkurvenverlaufs erfolgt, wobei beim Vorliegen einer Mutation in der Nukleinsäure-Sequenz sich die Schmelztemperatur einer Sonde und somit der Schmelzkurvenverlauf verändert, wobei die Schmelzkurvenanalyse wie oben beschrieben durchgeführt wird.

Die Erfindung wird in den nachfolgenden Ausführungsbeispielen näher erläutert.

Eine PCR-Reaktionsmischung bestehend aus DNA (200 U/10 $\mu$l) Kien Taq-Enzym (0,8 U/10 $\mu$l), 0,2 mM dNTP, PCR-Puffer und 0,2 $\mu$M Primer wurden 40 thermischen PCR-Zyklen (denaturierende Probe bei 94° C, Annealing bei 40° C) ausgesetzt und die zu analysierenden Schmelzkurven aus den Fluoreszenzsignalen generiert. Hierfür wurden fluoreszenzmarkierte Primersonden eingesetzt, die mit Fluoreszein markiert sind.

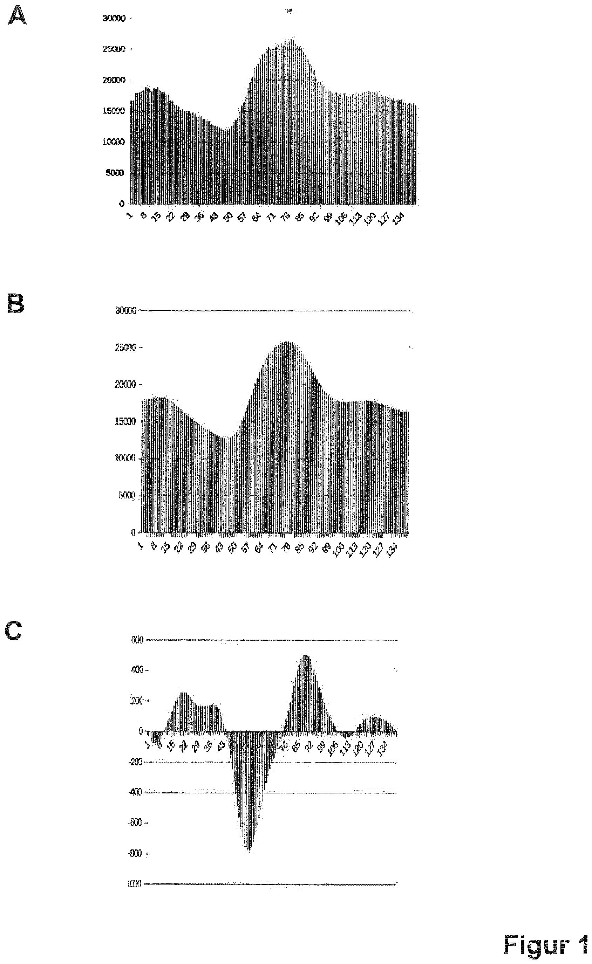

Die erhaltenen Schmelzkurvendaten wurden zunächst vorverarbeitet und so dargestellt, dass die relevanten Merkmale repräsentiert sind (Fig. 1A). Ferner wurden die Kurven mit einem Medianfilter geglättet, wobei ein Filter mit einem Fenster von fünf- und zehn pro Kurve angewendet wurde (Fig. 1B). Schließlich wurde aus der so vorverarbeiteten Kurve die negative Ableitung der geglätteten Kurve berechnet (Fig. 1C).

Für die Merkmalsextraktion wurden verschiedene Merkmalsvektoren für die verwendeten Klassifikatoren herangezogen, welche die relevanten Merkmale des Objektes beschreiben.

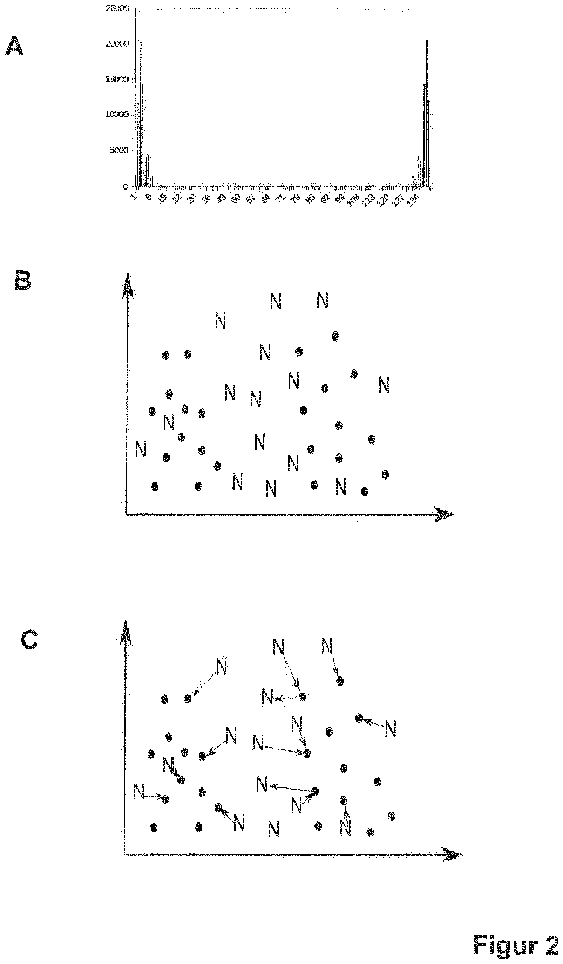

Mittels Fourieranalyse wurden die im Signal erhaltenen Frequenzanteile ermittelt. In Fig. 2A ist das Amplitudenspektrum gemäß den Fourier-Koeffizienten gezeigt. In diesem Beispiel sind Frequenzanteile des Spektrums zu sehen, jeweils 20 am Anfang und am Ende. Zur Darstellung wird eine komplexe Zahl verwendet, was bei der Verarbeitung zu einem Merkmalsvektor der Dimension 80 (40 komplexwertige Frequenzanteile; je 2 Zahlen als Realteil und Imaginärteil) führt.

Bei der Auswertung der einfachen Merkmale wurden die Peaks der Kurven analysiert, wobei drei positive Peaks (Maxima) und drei negative Peaks (Minima) bestimmt wurden. Die Maxima befinden sich mit einer Abweichung von fünf Kurvenpunkten bei 19, 90 und 28. Die Minima befinden sich bei 7,56 und 110, ebenfalls mit einer Abweichung von 5. Für die Merkmale wird bei den Maxima der Maximalwert der Intervalle bestimmt, bei den Minima der entsprechende Minimalwert. Zusätzlich werden 20 Harmonische der Fourieranalyse aufgenommen.

Die so extrahierten Merkmale einer Schmelzkurve werden mittels eines maschinellen Lernverfahrens analysiert. In dem hier gezeigten Beispiel erfolgt die Anwendung der lernenden Vektorquantisierung (LVQ), bei der die Merkmalsvektoren in eine Trainingsmenge und eine Testmenge aufgeteilt werden. Dabei werden Neuronen (Prototypen) im Merkmalsraum verteilt und an die Trainingsdaten, die in der Trainingsmenge enthalten sind, angepasst. Hier kommt die Distanzfunktion für die Trainingsdaten und Neuronen zur Anwendung. Pro Klasse werden mehrere Neuronen verwendet, wobei diese zur Initialisierung auf einen zufällig gewichteten Schwerpunkt der Merkmalsvektoren positioniert werden.

Für das "Training" wählt die LVQ einen Merkmalsvektor der Trainingsmenge und ermittelt das am nächsten gelegene Neuron. Stimmt die Klasse des Merkmalsvektors mit der Klasse des nächstgelegenen Neurons überein, wird das nächstgelegene Neuron in Richtung des Merkmalsvektors bewegt. Stimmt die Klasse des Merkmalsvektors nicht mit dem nächstgelegenen und nicht mit dem übernächstgelegenen Neuron überein, wird das nächstgelegene Neuron vom Merkmalsvektor weg bewegt. Dieser Vorgang wird mit unterschiedlichen Merkmalsvektoren mehrfach wiederholt. Die daraus entstandene Konfiguration der Neuronen wird temporär gespeichert.

Die Qualität des Trainings wird mit der Testmenge überprüft und das Training wird für verschiedene Initialisierungen wiederholt. Die Konfiguration der Neuronen für die beste Erkennung der Trainingsdaten wird gespeichert.

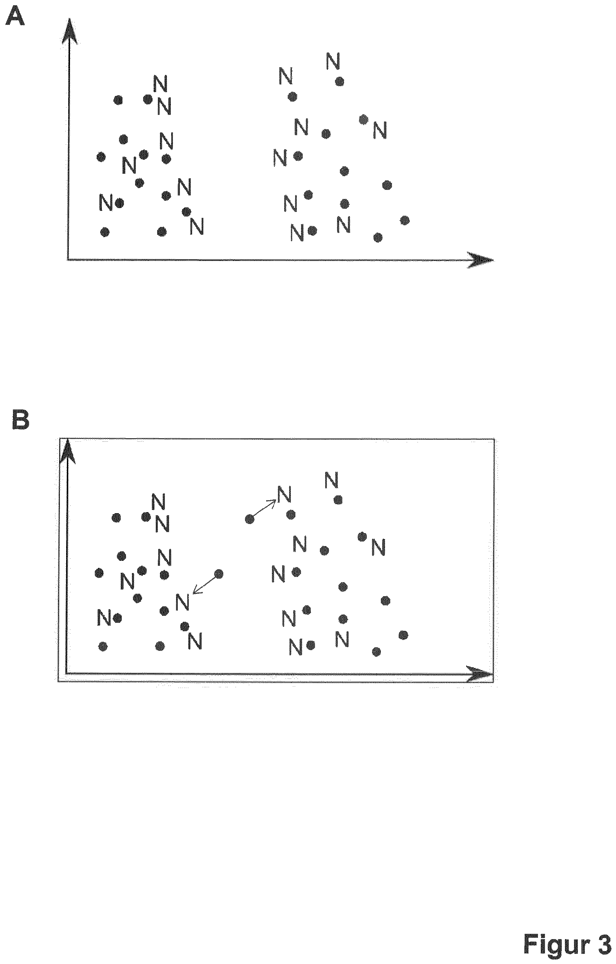

Dieser Zusammenhang ist in den Figuren 2B und 2C gezeigt. In der Figur 2B erkennt man die in einem Merkmalsraum verteilten Neuronen. Der Abstand eines jeden Neurons zu jedem Trainingsobjekt wird iterativ bestimmt. Gehören das aktuell betrachtete Trainingsobjekt und Neuron der gleichen Klasse an, so wird das Neuron in Richtung Trainingsobjekt verschoben, ansonsten in die Gegenrichtung bewegt (Fig. 2C). Die Größe der Verschiebungen kann mit einem Parameter festgesetzt werden. In Fig. 2C ist die iterative Neuronenverteilung im Rahmen des Trainings gezeigt. Nach einer bestimmten Anzahl von Iterationen erhält man ein angelerntes Netzwerk ohne eine finale Verteilung der Neuronen (Fig. 3A). Die Zuordnung eines unbekannten Objektes zu einer angelernten Klasse geschieht über die Ermittlung des kleinsten Abstandes der Objektmerkmale zu einem Neuron. Dies ist für die Klassifikation erforderlich. Das Neuron, welches dem Objekt am nächsten ist, bestimmt dessen Klassenzugehörigkeit (Fig. 3B).

Mittels einer nachgeschalteten Optimierung, beispielsweise über einen genetischen Algorithmus (GA), wird die Distanzfunktion an die Testmenge angepasst, wodurch die Erkennung der Kurven in der Testmenge verbessert wird. Dabei entspricht ein Lösungskandidat einem bestimmten Vektor d, welcher die Koeffizienten für die Distanzfunktion beinhaltet. Die Qualität eines Lösungskandidaten errechnet sich aus der richtig klassifizierten Anzahl der Merkmalsvektoren der Testmenge für die jeweilige Distanzfunktion. Lösungskandidaten mit hoher Qualität werden bevorzugt selektiert. Diese selektierten Lösungskandidaten werden gekreuzt oder mutiert. Daraus wird eine neue Population an Lösungskandidaten generiert. Bei der Kreuzung werden Elemente der Lösungskandidaten zufällig von dem einen oder anderen Lösungskandidaten übernommen. Bei der Mutation werden Elemente eines Lösungskandidaten zufällig verändert. Die Distanz berechnet sich für zwei Vektoren, einen Merkmalsvektor x und ein Neuron y für n Dimensionen mittels eines Faktors $d_i$ (Werte zwischen 0 und 1) durch

$$\frac{\sum_{i=1}^{n} j\, d_i (x_i - y_i)^2}{j}$$

Diese Distanzfunktion wird ebenfalls abgespeichert und dient mit der Konfiguration der Neuronen zur Erkennung neuer Kurven. Ist der Wert für $d_i$ für eine Komponente (Richtung) = 0, dann hat der Abstand von Objekt und Neuron in diese Richtung keinen Einfluss mehr.

Um das Klassifikationsergebnis zu evaluieren, wird die Unähnlichkeit eines Merkmalsvektors bestimmt. Die Unähnlichkeit berechnet sich aus dem Quotienten der durchschnittlichen Entfernung des Merkmalsvektors zu allen Neuronen zu einer zugeordneten klassifizierten Klasse und der Summe der durchschnittlichen Entfernung zu allen Prototypen der anderen Klassen. Je höher der Quotient ist, desto unähnlicher ist der Merkmalsvektor zu seiner Klasse.

**Patentansprüche**

1. Verfahren zum Nachweis und/oder Quantifizieren einer Ziel-Nukleinsäure in einer Polymerase-Kettenreaktion (PCR)-Probe, umfassend

- eine Ziel-Nukleinsäure mit einer zu amplifizierenden Nukleinsäure-Zielsequenz,
- eine thermostabile (DNA)-Polymerase,
- Desoxynucleosidtriphosphate (dNTPs),
- wenigstens ein zu der zu amplifizierenden Nukleinsäure-Zielsequenz komplementäres Oligonukleotid-Primerpaar, dessen Primer unter PCR-Bedingungen an den zu amplifizierenden Bereich der Nukleinsäure-Zielsequenz hybridisieren, wobei zunächst mehrere Amplifikationszyklen einer Polymerase-Kettenreaktion mit der PCR-Probe durchgeführt werden, wodurch Schmelzkurven erhalten werden, aus denen Signale generiert und einer Schmelzkurvenanalyse unterzogen werden, bei der:

- die Kurvenpunkte einer Schmelzkurve eingelesen und für jeden Kurvenpunkt die negative Steigung berechnet werden,
- die Schmelzkurven in ihre Spektralkomponenten zerlegt und die im Signal enthaltenen Frequenzanteile mittels Fourieranalyse für eine Merkmalsextraktion ermittelt werden,
- die extrahierten Merkmale einer Schmelzkurve mittels maschineller Lernverfahren unter Einsatz von Klassifikatoren analysiert und in Klassen eingeteilt werden, **dadurch gekennzeichnet, dass**
- zur Klasseneinteilung eine mathematische Optimierungsmethode angewendet wird, bei der zunächst bestimmte Merkmale der Kurve ermittelt werden, um Merkmalsvektoren zu bilden, wobei mehrere Merkmalsvektoren unabhängig voneinander von den eingesetzten Klassifikatoren wie Support Vector Maschine (SVM), Lernende Vektor-Quantisierung (LVQ) und Random Forest verwendet und bei der die eingesetzten Klassifikatoren unterschiedlich gewichtet werden, um eine Population von selektierbaren Lösungskandidaten zu generieren, mit der die Erkennung der Kurven in einer Testmenge verbessert wird, wobei über das dadurch erhaltene Ergebnis der Schmelzkurvenanalyse das Vorkommen und/oder die Menge der nachzuweisenden Ziel-Nukleinsäure in der Probe ermittelbar ist.

2. Verfahren nach Anspruch 1, **dadurch gekennzeichnet, dass** vor der Berechnung der negativen Steigungen der Kurvenpunkte eine Glättung der Daten durchgeführt wird.

3. Verfahren nach Anspruch 1 oder 2, **dadurch gekennzeichnet, dass** bei der Merkmalsextraktion eine Auswahl von Koeffizienten zur Bildung von Merkmalsvektoren getroffen wird.

4. Verfahren nach einem der Ansprüche 1 bis 3, **dadurch gekennzeichnet, dass** als Merkmalsvektoren Fourier-Koeffizienten, Wavelet-Koeffizienten und "einfache" Merkmale zum Einsatz kommen, und dass die extrahierten Merkmale einer Schmelzkurve mittels einer Support Vector Machine (SVM) und/oder einer lernenden Vektor-Quantisierung (LVQ) und/oder unter Einsatz der Random Forest-Methode klassifiziert werden.

5. Verfahren nach Anspruch 4, **dadurch gekennzeichnet, dass** bei Anwendung der Support Vector Machine (SVM) die Klassenzugehörigkeit y eines Merkmalsvektors x über eine Kernelfunktion ermittelbar ist durch:

$y = sgn(K(w,x)+b)$, wobei $K(w,x)$ = Kernelfunktion

6. Verfahren nach Anspruch 4 oder 5, **dadurch gekennzeichnet, dass** die SVM für die Schmelzkurvenanalyse angelernt wird, indem eine Menge von Testobjekten bereit gestellt wird, deren Klassenzugehörigkeit bekannt ist und deren Objekte nicht in der Trainingsmenge enthalten sind, wobei die Merkmale der Testobjekte extrahiert und der angelernten SVM übergeben werden, wodurch man die angenommene Klassenzugehörigkeit erhält, wobei der prozentuale Anteil der korrekt erkannten Testobjekte ein Maß für die Klassifikationsperformance darstellt.

7. Verfahren nach Anspruch 4, **dadurch gekennzeichnet, dass** die lernende Vektor-Quantisierung (LVQ) für die Schmelzkurvenanalyse angelernt wird, indem Neuronen in einem Merkmalsraum verteilt werden und nacheinander der Abstand eines jeden Neurons zu jedem Trainingsobjekt bestimmt wird, wobei bei gleicher Klassenzugehörigkeit das Neuron und das Trainingsobjekt aufeinander zubewegt, anderenfalls voneinander in Gegenrichtung weg bewegt werden, wodurch eine iterative Neuronenverteilung erhalten wird, wobei die Zuordnung eines unbekannten Objekts zu einer angelernten Klasse über die Ermittlung des kleinsten Abstands der Objektmerkmale zu einem Neuron erfolgt.

8. Verfahren nach Anspruch 4, **dadurch gekennzeichnet, dass** bei der lernenden Vektor-Quantisierung (LVQ) die Merkmalsvektoren in eine Trainingsmenge und in eine Testmenge mittels Distanzfunktion aufgeteilt werden, wobei die Distanzfunktion für zwei Vektoren, einen Merkmalsvektor x und ein Neuron y für n Dimensionen ermittelbar ist durch:

$$\sum_{i=1}^{N} d_i(x_i - y_i)^2$$

wobei die Zuordnung eines unbekannten Objektes zu einer angelernten Klasse über die Ermittlung des kleinsten Abstands der Objektmerkmale zu einem Neuron erfolgt, wobei das Neuron, welches dem Objekt am nächsten ist, dessen Klassenzugehörigkeit bestimmt.

9. Verfahren nach einem der Ansprüche 1 bis 8, **dadurch gekennzeichnet, dass** als mathematische Optimierungsmethode die Distanzfunktion mittels eines Genetischen Algorithmus zur Verbesserung der Kurvenerkennung an die Testmenge angepasst wird, indem die Qualität der Lösungskandidaten anhand der klassifizierten Anzahl der Merkmalsvektoren der Testmenge für die jeweilige Distanzfunktion bestimmt und die selektierten Lösungskandidaten gekreuzt oder mutiert werden, wodurch eine neue Population von Lösungskandidaten generiert wird.

10. Verfahren nach einem der Ansprüche 1 bis 9, **dadurch gekennzeichnet, dass** nach Anwendung der mathematischen Optimierungsmethode das Klassifikationsergebnis durch Bestimmung der Unähnlichkeit der Merkmalsvektoren ermittelt wird, welche sich aus dem Quotienten der durchschnittlichen Entfernung des Merkmalsvektors zu allen Neuronen seiner zugeordneten Klasse und der Summe der durchschnittlichen Entfernungen zu allen Prototypen der anderen Klassen ergibt, wobei ein höherer Quotient auf eine höhere Unähnlichkeit des Merkmalsvektors zu seiner Klasse hinweist.

11. Verfahren nach einem der Ansprüche 1 bis 10, **dadurch gekennzeichnet, dass** das Oligonukleotid-Primerpaar einen Überschuss-Primer und einen limitierenden Primer bei der Amplifikation umfasst, wobei die Konzentration des limitierenden Primers so gewählt ist, dass er nach den ersten Amplifikationszyklen im Vergleich zum Überschuss-Primer erschöpft ist und wobei die Schmelztemperatur des limitierenden Primers höher ist als die Schmelztemperatur des Überschuss-Primers.

12. Testsystem zur Durchführung einer symmetrischen oder asymmetrischen Polymerase-Kettenreaktion (PCR) für den Nachweis einer Ziel-Nukleinsäure in einer Probe, umfassend

   - eine Ziel-Nukleinsäure mit einer zu amplifizierenden Nukleinsäure-Zielsequenz,
   - eine thermostabile (DNA)-Polymerase,
   - Desoxynucleosidtriphosphate (dNTPs),
   - wenigstens ein zu der zu amplifizierenden Nukleinsäure-Zielsequenz komplementäres Oligonukleotid-Primerpaar, dessen Primer unter PCR-Bedingungen an den zu amplifizierenden Bereich der Nukleinsäure-Zielsequenz hybridisieren,
   - eine Datenerfassungseinheit zur Erfassung der über die Amplifikationsreaktionen bei der PCR erhaltenen Schmelzkurven,
   - eine Analyseeinheit zur Schmelzkurvenanalyse der erhaltenen Schmelzkurven gemäß einem in den Ansprüchen 1 bis 11 beschriebenen Verfahren.

13. Testsystem nach Anspruch 12, **dadurch gekennzeichnet, dass** bei der PCR-Reaktion Nukleinsäure-Sonden zum Einsatz kommen, die ein Reporter- und/oder Quencher-Molekül umfassen.

14. Verwendung eines Test-Systems gemäß einem der Ansprüche 12 oder 13 zur Durchführung einer Multiplex-PCR oder Reverse Transkriptase (RT)-PCR oder quantitativen-(Multiplex)-Echtzeit-PCR (qPCR) oder (Multiplex)-LATE-PCR.

15. Verfahren zum Nachweis von Mutationen in einer Nukleinsäure-Sequenz, bei dem eine Probe in Anwesenheit von zu einem Bereich der Nukleinsäure-Sequenz spezifischen Sonden mit einer bereits amplifizierten Nukleinsäure-Zielsequenz erhitzt und anschließend schrittweise abgekühlt wird, während eine Erfassung des Schmelzkurvenverlaufs erfolgt, wobei beim Vorliegen einer Mutation in der Nukleinsäure-Sequenz sich die Schmelztemperatur einer Sonde und somit der Schmelzkurvenverlauf verändert, wobei die Schmelzkurvenanalyse gemäß einem in den Ansprüchen 1 bis 11 beschriebenen Verfahren durchgeführt wird.

**Claims**

1. Method for detecting and/or quantifying a target nucleic acid in a polymerase chain reaction (PCR) sample, comprising:

   - a target nucleic acid having a nucleic acid target sequence to be amplified,
   - a thermostable (DNA) polymerase,
   - deoxynucleoside triphosphates (dNTPs)
   - at least one oligonucleotide primer pair that is complementary to the nucleic acid target sequence to be amplified, the primers of which primer pair, under PCR conditions, hybridise in the region to be amplified of the nucleic acid target sequence, initially a plurality of amplification cycles of a polymerase chain reaction being carried out using the PCR sample, as a result of which melting curves are obtained, from which signals are generated and undergo a melting curve analysis in which

- the curve points of a melting curve are read and the negative gradient is calculated for each curve point,
- the melting curves are broken down into their spectral components and the frequency components contained in the signal are ascertained by means of Fourier analysis for a feature extraction,
- the extracted features of a melting curve are analysed by means of machine learning methods using classifiers and are divided into classes, **characterised in that**,
- for the division into classes, a mathematical optimisation method is applied in which determined features of the curve are initially ascertained in order to form feature vectors, a plurality of feature vectors being used independently of one another by the utilised classifiers such as a support vector machine (SVM), learning vector quantisation (LVQ) and random forest, and in which the utilised classifiers are weighted differently, in order to generate a population of selectable solution candidates, by means of which identification of the curves in the test batch is improved, it being possible to ascertain the occurrence and/or the amount of target nucleic acid to be detected in the sample by means of the result of the melting curve analysis obtained thereby.

2. Method according to claim 1, **characterised in that** the data are smoothed before the negative gradients of the curve points are calculated.

3. Method according to either claim 1 or claim 2, **characterised in that**, during the feature extraction, coefficients for forming feature vectors are selected.

4. Method according to any of claims 1 to 3, **characterised in that** Fourier coefficients, wavelet coefficients and "simple" features are used as feature vectors, and **in that** the extracted features of a melting curve are classified by means of a support vector machine (SVM) and/or learning vector quantisation (LVQ) and/or by using the random forest method.

5. Method according to claim 4, **characterised in that**, when applying the support vector machine (SVM), the class association y of a feature vector x can be ascertained by a kernel function, by:

y = sgn(K(w,x)+b), where K(w,x) = kernel function

6. Method according to either claim 4 or claim 5, **characterised in that** the SVM for the melting curve analysis is learned by means of a quantity of test objects being provided, the class association of which is known and the objects of which are not contained in the training quantity, the features of the test objects being extracted and the learned SVM being transferred, as a result of which the assumed class association is obtained, the percentage of correctly identified test objects being an indication of classification performance.

7. Method according to claim 4, **characterised in that** the learning vector quantisation (LVQ) for the melting curve analysis is learned by means of neurons being distributed in a feature space and the distance between each neuron and each training object being successively determined, the neuron and the training object being moved towards one another in the case of the same class association and being moved away from one another in the opposite direction if this is not the case, as a result of which an iterative distribution of neurons is obtained, an unknown object being allocated to a learned class by ascertaining the smallest distance between the object features and a neuron.

8. Method according to claim 4, **characterised in that**, in the learning vector quantisation (LVQ), the feature vectors are split up by means of distance function into a training quantity and into a test quantity, it being possible for the distance function for two vectors, one feature vector x and one neuron y, to be determined for n dimensions by:

$$\sum_{i=1}^{N} d_i(x_i - y_i)^2$$

an unknown object being allocated to a learned class by means of ascertaining the smallest distance between the object features and a neuron, the neuron which is closest to the object determining the class association thereof.

9. Method according to any of claims 1 to 8, **characterised in that**, as a mathematical optimisation method, the distance function is adapted to the test quantity by means of a genetic algorithm for improving curve identification by means of the quality of the solution candidates being determined for the relevant distance function on the basis of the classified number of feature vectors of the test quantity, and the selected solution candidates being hybridised or mutated, as a result of which a new population of solution candidates is generated.

**10.** Method according to any of claims 1 to 9, **characterised in that**, after applying the mathematical optimisation method, the classification result is ascertained by determining the dissimilarity between the feature vectors, which dissimilarity is found from the quotients of the average spacing between the feature vector and all the neutrons of its associated class, and the sum of the average spacing from all prototypes of the other class, a higher quotient indicating a higher level of dissimilarity between the feature vector and its class.

**11.** Method according to any of claims 1 to 10, **characterised in that** the oligonucleotide primer pair comprises an excess primer and a limiting primer during amplification, the concentration of the limiting primer being selected such that, after the first amplification cycles, said limiting primer is exhausted in comparison to the excess primer, and the melting temperature of the limiting primer being higher than the melting temperature of the excess primer.

**12.** Test system for carrying out a symmetric or asymmetric polymerase chain reaction (PCR) for detecting a target nucleic acid in a sample, said system comprising:

- a target nucleic acid having a nucleic acid target sequence to be amplified,
- a thermostable (DNA) polymerase,
- deoxynucleoside triphosphates (dNTPs),
- at least one oligonucleotide primer pair that is complementary to the nucleic acid target sequence to be amplified, the primers of which primer pair, under PCR conditions, hybridise in the region to be amplified of the nucleic acid target sequence,
- a data recording unit for recording the melting curves obtained by the amplification reactions during the PCR,
- an analysis unit for melting curve analysis of the obtained melting curves according to a method described in claims 1 to 11.

**13.** Test system according to claim 12, **characterised in that**, during the PCR reaction, nucleic acid probes are used that comprise a reporter molecule and/or quencher molecule.

**14.** Use of a test system according to either claim 12 or claim 13 for carrying out a multiplex PCR or reverse transcriptase (RT)-PCR or quantitative (multiplex) real-time PCR (qPCR) or (multiplex) LATE PCR.

**15.** Method for detecting mutations in a nucleic acid sequence, in which method a sample is heated in the presence of probes which are specific to a region of the nucleic acid sequence and have an already amplified nucleic acid target sequence, and is subsequently cooled in stages, while the melting curve progression is recorded, wherein, if there is a mutation in the nucleic acid sequence, the melting temperature of a probe, and thus the melting curve progression, changes, wherein the melting curve analysis is carried out according to a method described in claims 1 to 11.

**Revendications**

**1.** Procédé destiné à la détection et/ou à la quantification d'un acide nucléique cible dans une sonde de réaction de polymérisation en chaîne (PCR), comprenant

- un acide nucléique cible avec une séquence cible d'acide nucléique à amplifier,
- une ADN-polymérase thermostable,
- des désoxynucléotides triphosphates (dNTP),
- au moins une paire d'amorces oligonucléotidiques complémentaires à la séquence cible d'acide nucléique à amplifier, dont les amorces s'hybrident dans des conditions de PCR au niveau de la région de la séquence cible d'acide nucléique à amplifier, où plusieurs cycles d'amplification d'une réaction de polymérisation en chaîne sont tout d'abord réalisés avec la sonde de PCR, grâce à quoi des courbes de fusion sont obtenues, à partir desquelles des signaux sont générés et sont soumis à une analyse de courbe de fusion, où :

- les points de courbe d'une courbe de fusion sont lus et la pente négative est calculée pour chaque point de courbe,
- les courbes de fusion sont décomposées en leurs composantes spectrales et les composantes de fréquence contenues dans le signal sont déterminées au moyen d'une analyse de Fourier pour une extraction de caractéristiques,
- les caractéristiques extraites d'une courbe de fusion sont analysées par un procédé d'apprentissage automatique à l'aide de classificateurs et sont classées en catégories, **caractérisé en ce que**
- pour la classification, une méthode d'optimisation mathématique est employée, lors de laquelle certaines caractéristiques de la courbe sont tout d'abord déterminées, pour former des vecteurs de caractéristiques, où plusieurs vecteurs de caractéristiques sont utilisés indépendamment les uns des autres par les classificateurs utilisés tels qu'une machine à vecteurs de support (SVM), une quantification vectorielle adaptative (LVQ) et une forêt d'arbres décisionnels et où les classificateurs utilisés sont pondérés différemment, pour générer une population de solutions candidates sélectionnables, avec lesquelles la reconnaissance des courbes est améliorée dans un ensemble d'essai, où, grâce au résultat ainsi obtenu de l'analyse de courbe de fusion, la présence et/ou la quantité de l'acide nucléique cible à détecter dans la sonde peut être déterminée.

2. Procédé selon la revendication 1, **caractérisé en ce que**, avant le calcul des pentes négatives des points de la courbe, un lissage des données est effectué.

3. Procédé selon la revendication 1 ou 2, **caractérisé en ce que**, lors de l'extraction de caractéristiques, une sélection de coefficients pour la formation de vecteurs de caractéristiques est effectuée.

4. Procédé selon l'une quelconque des revendications 1 à 3, **caractérisé en ce que** des coefficients de Fourier, des coefficients d'ondelettes et des caractéristiques « simples » sont utilisés à titre de vecteurs de caractéristiques, et **en ce que** les caractéristiques extraites d'une courbe de fusion sont classées à l'aide d'une machine à vecteurs de support (SVM) et/ou d'une quantification vectorielle adaptative (LVQ) et/ou en utilisant une méthode de forêt d'arbres décisionnels.

5. Procédé selon la revendication 4, **caractérisé en ce que**, lorsque la machine à vecteurs de support (SVM) est utilisée, la catégorie y d'un vecteur de caractéristique x peut être déterminée au moyen d'une fonction noyau, comme suit :

y = sgn(K(w,x)+b), où K(w,x) = fonction noyau.

6. Procédé selon la revendication 4 ou 5, **caractérisé en ce que** la machine SVM est entraînée à l'analyse de courbe de fusion, en fournissant un ensemble d'objets d'essai dont la catégorie est connue et dont les objets ne sont pas inclus dans l'ensemble d'entraînement, où les caractéristiques des objets d'essai sont extraites et transmises à la SVM entraînée, grâce à quoi la catégorie supposée est obtenue, où le pourcentage des objets d'essais correctement reconnus est une mesure de la performance de classification.

7. Procédé selon la revendication 4, **caractérisé en ce que** la quantification vectorielle adaptative (LVQ) est entraînée à l'analyse de courbe de fusion, en classant des neurones dans un espace de caractéristique puis en déterminant la distance entre chaque neurone et chaque objet d'entraînement, où pour une même catégorie, le neurone et l'objet d'entraînement sont déplacés l'un vers l'autre, sinon ils sont éloignés l'un de l'autre dans le sens contraire, grâce à quoi une classification de neurones itérative est obtenue, où l'assignation d'un objet inconnu à une catégorie définie par entraînement est réalisée grâce à la détermination de la plus petite distance entre la caractéristique d'objet et un neurone.

8. Procédé selon la revendication 4, **caractérisé en ce que** lors de la quantification vectorielle adaptative (LVQ), les vecteurs de caractéristiques sont classés en un ensemble d'entraînement et un ensemble d'essai au moyen de la fonction de distance, où la fonction de distance pour deux vecteurs, un vecteur de caractéristique x et un neurone y pour n dimensions peut être déterminée comme suit :

$$\sum_{i=1}^{N} d_i(x_i - y_i)^2$$

où l'assignation d'un objet inconnu à une catégorie définie par entraînement est réalisée grâce à la détermination de la plus petite distance entre la caractéristique d'objet et un neurone, où le neurone, lequel est le plus proche de l'objet, détermine la catégorie de ce dernier.

9. Procédé selon l'une quelconque des revendications 1 à 8, **caractérisé en ce que** en tant que méthode d'optimisation mathématique, la fonction de distance est adaptée au moyen d'un algorithme génétique pour améliorer la reconnaissance de courbe au niveau l'ensemble d'essai en déterminant la qualité de la solution candidate à l'aide du nombre classé de vecteurs de caractéristiques de l'ensemble d'essai pour la fonction de distance respective et en croisant ou en mutant les solutions candidates sélectionnées, grâce à quoi une nouvelle population de solutions candidates est générée.

10. Procédé selon l'une quelconque des revendications 1 à 9, **caractérisé en ce que**, après application de la méthode d'optimisation mathématique, le résultat de classification est déterminé en déterminant la dissemblance des vecteurs de caractéristiques, laquelle est le quotient de la distance moyenne du vecteur de caractéristique à tous les neurones de sa catégorie assignée et de la somme des distances moyennes à tous les prototypes des autres catégories, où un quotient plus élevé indique une plus grande dissemblance du vecteur de caractéristique par rapport à sa catégorie.

11. Procédé selon l'une quelconque des revendications 1 à 10, **caractérisé en ce que** la paire d'amorces oligonucléotidiques comprend une amorce en excès et une amorce limitante lors de l'amplification, où la concentration de l'amorce limitante est choisie de sorte qu'elle soit épuisée après les premiers cycles d'amplification par rapport à l'amorce en excès et où la température de fusion de l'amorce limitante est supérieure à la température de fusion de l'amorce en excès.

12. Système d'essai destiné à réaliser une réaction de polymérisation en chaîne (PCR) symétrique ou asymétrique pour la détection d'un acide nucléique cible dans une sonde, comprenant

   - un acide nucléique cible avec une séquence cible d'acide nucléique à amplifier,
   - une ADN polymérase thermostable,
   - des désoxynucléotides triphosphates (dNTP),
   - au moins une paire d'amorces oligonucléotidiques complémentaires à la séquence cible d'acide nucléique à amplifier, dont les amorces s'hybrident dans des conditions de PCR au niveau de la région de la séquence cible d'acide nucléique à amplifier,
   - une unité d'acquisition de données destinée à l'acquisition des courbes de fusion obtenues par les réactions d'amplification lors de la PCR,
   - une unité d'analyse pour l'analyse de courbe de fusion des courbes de fusion obtenues conformément au procédé décrit selon l'une quelconque des revendications 1 à 11.

13. Système d'essai selon la revendication 12, **caractérisé en ce que**, lors de la réaction de PCR, des sondes d'acide nucléique sont utilisées, lesquelles comprennent une molécule rapporteur et/ou une molécule désactivateur.

14. Utilisation d'un système d'essai selon l'une quelconque des revendications 12 ou 13 pour effectuer une PCR multiplexe ou une PCR de transcriptase inverse (RT-PCR) ou une PCR en temps réel (multiplexe) quantitative (qPCR) ou une PCR LATE(multiplexe).

15. Procédé de détection de mutations dans une séquence d'acide nucléique, dans lequel une sonde est chauffée en présence de sondes spécifiques d'une région de la séquence d'acide nucléique avec une séquence cible d'acide nucléique déjà amplifiée puis est refroidie progressivement, pendant qu'une acquisition de tracé de courbe de fusion a lieu, où en présence d'une mutation dans la séquence d'acide nucléique, la température de fusion d'une sonde et donc le tracé de courbe de fusion changent, où l'analyse de courbe de fusion est réalisée conformément au procédé décrit selon l'une quelconque des revendications 1 à 11.

Figur 1

A

B

C

**Figur 2**

A

B

**Figur 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1375674 B1
- EP 1942196 B1
- DE 102007041864 B4
- DE 102007031137 A1
- EP 1288314 B1
- US 5273632 A
- US 5748491 A
- US 5346306 A
- US 316614 P
- WO 2014022827 A

**In der Beschreibung aufgeführte Nicht-Patentliteratur**

- **MULIS K et al.** Specific enzymatic amplification of DNA in vitro: the polmerase chain reaction. *Cold Spring Harb Symp Quant Biol,* 1986, vol. 51, 263-273
- **HENEGARIU et al.** *Multiplex PCR: critical parameters and step-by-step protocol,* 1997, vol. 23, 504-511
- **MOTHERSHED EA ; WHITNEY AM.** Nucleic acid-based methods for the detection of bacterial pathogenes: present and future considerations for the clinical laboratory. *Clin Chim Acta,* 2006, vol. 363, 206-220
- **HEID CA et al.** *Real time quantitative PCR,* 1996, vol. 6, 986-994
- **ESPY MJ et al.** *Real-time PCR in clinical microbiology: applications for routine laboratory testing,* 2006, vol. 19, 165-256
- **WITTWER CT et al.** Real-time multiplex PCR assays. *Methods,* 2001, vol. 25, 430-442
- **MACKAY IM et al.** Real-time PCR in virology. *Nucleic Acids res,* 2002, vol. 30, 1292-1305
- **HARTSHORN C et al.** Single-cell duplex RT-LATE-PCR reveals Oct4 and Xist RNA gradients in 8-cell embryos. *BMC Biotechnol.,* 2007, vol. 7, 87
- **GYLLENSTEN UB ; ERLICH HA.** Generation of single-stranded DNA by the polymerase chain reaction and ist application to direct sequencing oft he HLA-DQA locus. *Proc Natl Acad Sci U S A,* 1988, vol. 85, 7652-7656
- **SZILVÁSI A. et al.** Asymmetric PCR increases efficiency of melting peak analysis on the LightCycler. *Clin Biochem,* 2005, vol. 38, 727-730
- **SANCHEZ JA et al.** Linear-after-the-exponential (LATE)-PCR: an advanced method of asymmetric PCR and its uses in quantitative real-time analysis. *Proc Natl Acad Sci U S A,* 2004, vol. 101, 1933-1938
- **SANCHEZ JA et al.** Two-temperature LATE- PCR endpoint genotyping. *BMC Biotechnol,* 2006, vol. 6, 44